# FASCICULE DE BREVET EUROPEEN

(11) **EP 1 906 834 B1**
(45) Date de publication et mention de la délivrance du brevet: **18.07.2012**
(21) Numéro de dépôt: 06764835.2
(22) Date de dépôt: 02.06.2006
(51) Int. Cl.: A61B 6/00

(54) **TETE D'IMAGERIE ET DE TRAITEMENT D'ORGANES D'ETRES VIVANTS ET PROCEDE DE FABRICATION**
KOPF ZUR BILDLICHEN DARSTELLUNG UND BEHANDLUNG VON ORGANEN EINES LEBEWESENS UND VERFAHREN ZUR HERSTELLUNG DAVON
HEAD FOR IMAGING AND TREATING ORGANS OF LIVING BEINGS AND METHOD FOR MAKING SAME

(30) Priorité: 03.06.2005 FR 0551503
(43) Date de publication de la demande: 09.04.2008
(73) Titulaire: Theraclion, 75019 Paris (FR)
(72) Inventeur: LACOSTE, François, F-75015 Paris (FR)
(74) Mandataire: CAPRI
(86) Numéro de dépôt international: PCT/FR2006/050512
(87) Numéro de publication internationale: WO 2006/129045

(56) Documents cités:
- EP-A- 0 400 196
- EP-A2- 1 805 849
- DE-A1- 4 205 030
- DE-C2- 3 844 672
- FR-A- 2 827 149
- US-A- 6 071 238

## Description

La présente invention concerne une tête d'imagerie et de traitement d'organes ou de tissus d'êtres vivants, notamment utile pour le traitement de diverses tumeurs, comme par exemple les tumeurs thyroïdiennes, du sein ou de l'utérus. La présente invention concerne également un procédé de fabrication d'une pièce constitutive de la tête. La présente invention concerne par conséquent le domaine des dispositifs de thérapie, et plus particulièrement les dispositifs de thérapie sous contrôle ultrasonore et plus particulièrement les dispositifs de thérapie par ultrasons de puissance. Le traitement par ultrasons de puissance focalisés est connu sous l'acronyme HIFU (High Intensity Focused Ultrasound)

La tête de traitement et d'imagerie de l'invention est destinée à être montée sur un bras pourvu de moteurs, afin de pouvoir déplacer la tête de manière précise dans différentes directions. La tête est également reliée à une armoire électronique de commande et de traitement, ainsi qu'à un scanner, par exemple ultrasonique. La tête est d'autre part généralement reliée à une unité de refroidissement, qui permet de faire circuler un milieu de propagation réfrigéré à l'intérieur de la tête. Il s'agit là d'une conception relativement conventionnelle pour une tête de traitement et d'imagerie ultrasonique, utilisant par exemple un traitement HIFU et une sonde d'imagerie échographique. Il ne s'agit là que d'un type particulier de dispositif de traitement : il existe bien entendu d'autres types de dispositif de traitement, qui utilisent d'autres techniques d'imagerie et de traitement, sans pour autant sortir du cadre de l'invention.

Le traitement réalisé par les moyens de traitement de la tête peut utiliser des ultrasons, focalisés ou non. Parmi les traitements par ultrasons focalisés, on connaît déjà le traitement par HIFU (High Intensity Focused Ultrasound). L'invention utilisera de préférence, mais pas exclusivement, ce type de traitement HIFU. En effet, d'autres types de traitement peuvent être utilisés dans le cadre de la présente invention, et notamment tout traitement utilisant des ondes ou des rayonnements susceptibles d'atteindre une cible située dans un organe ou un tissu d'un être vivant. Le traitement de la présente invention est de préférence non invasif : toutefois, un traitement invasif peut également être envisagé dans le cadre de la présente invention.

La tête d'imagerie et de traitement, outre ses moyens de traitement, comprend également des moyens d'imagerie qui peuvent être de toute nature, comme par exemple une imagerie par sonde échographique, par sonde à rayon X, ou par IRM. Une imagerie par échographie ultrasonique sera cependant préférée dans le cadre de la présente invention.

Il existe déjà de telles têtes d'imagerie et de traitement qui permettent de combiner en une seule unité les moyens de traitement et les moyens d'imagerie nécessaires pour localiser et représenter la cible à traiter. Il est en effet indispensable que la cible à traiter soit parfaitement localisée pour ne pas endommager des parties saines du tissu ou de l'organe à traiter. Pour cela, les moyens d'imagerie doivent délivrer une représentation fiable et précise du site de la cible. Le document EP 0400196 décrit un système selon le préambule de la revendication 1.

Pour permettre cette localisation précise de la cible à traiter, il est indispensable que les moyens de traitement et les moyens d'imagerie soient positionnés mutuellement les uns par rapport aux autres de manière parfaitement précise. Il est tout d'abord possible de mettre en place des moyens d'imagerie, afin qu'il prélève une image précise du site à traiter. Les moyens d'imagerie peuvent ensuite être écartés et les moyens de traitement sont alors mis en place et positionnés de manière très précise, afin qu'ils correspondent avec l'image prise par les moyens d'imagerie. Une autre technique est de coupler mécaniquement les moyens d'imagerie et les moyens de traitement sur une même et unique tête en des endroits précis. Les moyens d'imagerie, par exemple une sonde échographique, sont montés sur la tête dans un logement approprié ou fixés en place par collage. Les moyens d'imagerie sont alors intégrés à la tête. Avec une telle tête, le type de moyen d'imagerie est imposé par la tête, étant donné que les moyens d'imagerie sont collés à la tête ou installés dans un logement spécifique à ces moyens. On ne peut donc, pas utiliser d'autres moyens d'imagerie qui seraient peut-être plus appropriés pour telle ou telle utilisation ou tel ou tel site à traiter. Par exemple selon les organes à traiter, on préférera une sonde échographique à barrette courbe ou linéaire, ou fonctionnant selon une gamme de fréquences déterminée.

La présente invention a pour but de surmonter les inconvénients susmentionnés de l'art antérieur en définissant une tête d'imagerie et de traitement plus modulable, sans pour autant négliger le positionnement précis des moyens d'imagerie par rapport aux moyens de traitement.

Pour atteindre ce but, la présente invention propose que les moyens d'imagerie soient montés de manière amovible sur un adaptateur assurant le positionnement des moyens d'imagerie relativement aux moyens de traitement. La tête comporte un corps de montage comprenant une zone de fixation des moyens de traitement et un logement de montage des moyens d'imagerie, ledit logement de montage recevant ledit adaptateur dans lequel les moyens d'imagerie sont montés de manière amovible. C'est donc l'adaptateur qui permet de positionner avec précision les moyens d'imagerie par rapport au corps de montage de la tête. L'adaptateur constitue ainsi une pièce intermédiaire ou de transition, qui tient compte d'une part de la forme du logement de montage du corps, et d'autre part de la forme des moyens d'imagerie. L'adaptateur permet ainsi de coupler avec précision les moyens d'imagerie à la tête sur laquelle sont montés également les moyens de traitement. Toutefois, l'adaptateur permet de rendre les moyens d'imagerie indépendants de la tête en ce qui concerne les formes respectives des moyens d'imagerie et du logement de montage, puisque c'est l'adaptateur qui permet de les relier ensemble. En ce sens, l'adaptateur permet de découpler les moyens d'imagerie de la tête. Par conséquent, en utilisant un adaptateur d'un autre type, qui est adapté à un autre type de moyens d'imagerie, on peut monter de manière précise et amovible ces autres moyens d'imagerie sur la tête de l'invention. Par conséquent, en prévoyant un jeu d'adaptateurs compatibles avec une large gamme de moyens d'imagerie, on peut monter n'importe quels moyens d'imagerie sur la tête de l'invention.

Selon une forme de réalisation avantageuse, la tête comprend un espace de propagation contenant un milieu de propagation apte à transmettre les ondes ou rayonnements émis(es) par les moyens d'imagerie et/ou les moyens de traitement, le corps formant une fenêtre qui fait communiquer le logement avec l'espace de propagation, lesdits moyens d'imagerie comportant une partie apicale et une partie basale, ladite partie apicale incorporant une face d'émission/réception d'ondes ou de rayonnement, reçue dans l'adaptateur de manière à émettre à travers la fenêtre dans l'espace de propagation. De préférence, l'adaptateur comporte une paroi frontale positionnée devant la face d'émission/réception des moyens d'imagerie et obturant au moins partiellement la fenêtre, ladite paroi frontale formant une lentille divergente, apte à éloigner le point focal des ondes ou rayonnements émis à travers la lentille et l'espace de propagation. On se sert ainsi de l'adaptateur pour déplacer le point focal des moyens d'imagerie. Ceci est particulièrement intéressant lorsque l'on utilise une sonde échographique dont la face d'émission/réception d'ondes est normalement destinée à venir en contact direct de la peau du patient. Toutefois, dans une tête d'imagerie et de traitement, la face d'émission/réception de la sonde échographique n'est pas en contact de la peau du patient, mais décalée d'une distance d'environ 3 cm. C'est pourquoi il est nécessaire d'éloigner le point focal des ondes émises par la sonde échographique d'une distance d'environ 3 cm. Dans la présente invention, on se sert de l'adaptateur pour réaliser cette lentille divergente qui permet de décaler le point focal de la sonde échographique d'une distance de l'ordre de quelques centimètres, de préférence 3 cm. Entre la face d'émission/réception de la sonde et la peau du patient, les ondes se propagent à travers l'espace de propagation qui est rempli d'un milieu de propagation qui est conventionnellement de l'eau.

Selon l'invention, l'adaptateur comprend une bague d'étanchéité, assurant l'étanchéité entre les moyens d'imagerie et le logement de montage, les moyens d'imagerie déformant avantageusement la bague lors de leur insertion dans la bague. Ainsi, l'adaptateur, en plus de sa fonction de positionnement des moyens de traitement, assure également une fonction d'étanchéité qui est avantageusement améliorée par la déformation de la bague de manière à créer un contact intime entre la bague et le logement de montage, et entre la bague et les moyens d'imagerie. L'adaptateur joue ainsi un rôle de joint d'étanchéité. Avantageusement, l'adaptateur est en matériau élastomère.

La bague d'étanchéité peut être combinée à la lentille divergente ou non. Dans ce dernier cas, la face d'émission/réception des moyens d'imagerie est alors en contact direct avec le milieu de propagation de l'espace de propagation.

Selon une autre caractéristique de l'invention, les moyens d'imagerie sont maintenus en position dans ledit adaptateur au moyen d'un système de maintien apte à solliciter les moyens d'imagerie dans ledit adaptateur. Ce système de maintien pousse donc les moyens d'imagerie contre l'adaptateur, qui est ainsi coincé entre les moyens d'imagerie et le logement de montage du corps. Ce système de maintien permet ainsi de bloquer les moyens d'imagerie dans l'adaptateur, ce qui assure un positionnement précis de la face d'émission/réception des moyens d'imagerie par rapport aux moyens de traitement. De plus, la force de poussée créée par le système de maintien améliore l'étanchéité au niveau de l'adaptateur.

Selon une forme de réalisation avantageuse, les moyens d'imagerie comportent une sonde échographique à barrettes linéaires. De préférence, le corps forme une fenêtre allongée, les barrettes linéaires étant positionnées pour émettre à travers la fenêtre, les moyens de traitement étant positionnés de part et d'autre de la fenêtre allongée. L'utilisation d'une sonde échographique à barrettes linéaires, disposées de manière médiane en coupant les moyens de traitement en deux parties sensiblement identiques, est une caractéristique qui peut être protégée indépendamment du fait que les moyens d'imagerie sont montés de manière amovible sur un adaptateur. En d'autres termes, cette caractéristique peut être protégée indépendamment.

D'autre part, les moyens de traitement peuvent comporter un transducteur piézo-électrique focalisé du type HIFU. Ainsi, à la fois les moyens d'imagerie et les moyens de traitement utilisent des ultrasons, de sorte que l'espace de propagation peut être rempli d'un milieu de propagation approprié à la fois pour les moyens d'imagerie et les moyens de traitement.

L'invention a également pour objet un procédé de fabrication d'un adaptateur pour des moyens d'imagerie incorporés dans une tête d'imagerie et de traitement focalisés selon l'une quelconque des revendications précédentes, dans lequel ledit procédé comprend les étapes de:
(a) fournir des moyens d'imagerie comportant une partie apicale comprenant une face d'émission servant à la transmission d'ondes ou de rayonnement,
(b) numériser ladite paroi apicale des moyens d'imagerie afin d'obtenir une représentation géométrique et une estimation précise des dimensions de cette partie apicale,
(c) fournir un moule présentant une géométrie et des dimensions en accord avec le résultat obtenu à l'étape (b),
(d) couler un ou plusieurs matériaux élastomériques dans le moule, de manière à obtenir un produit de moulage formant un adaptateur apte à parfaitement se positionner sur la partie apicale des moyens d'imagerie.

Avantageusement, le moule est réalisé avec des dimensions légèrement plus petites que la partie apicale, de façon que l'adaptateur soit naturellement comprimé lorsque le moyen d'imagerie est inséré dans la tête.

L'invention sera maintenant plus amplement décrite en référence aux dessins joints, donnant à titre d'exemples non limitatifs plusieurs modes de réalisation de l'invention.
La figure 1 est une vue en coupe transversale verticale, à travers une tête de traitement et d'imagerie selon une première forme de réalisation de l'invention,
La figure 2 est une vue similaire à celle de la figure 1, selon un plan de coupe verticale décalée de 90° par rapport à celui de la figure 1,
La figure 3 est une vue de dessous de la tête des figures 1 et 2, avec le ballonnet retiré,
La figure 4 est une vue partielle en section transversale d'une tête de traitement selon une seconde forme de réalisation de l'invention,
La figure 5 est une vue similaire à celle de la figure 4, pour une tête de traitement de l'art antérieur,
La figure 6 est une vue partielle similaire à celle de la figure 1, pour un troisième mode de réalisation de l'invention,
La figure 7 est une vue partielle similaire à celle de la figure 2 de la tête de la figure 6, et
La figure 8 est une vue schématique en perspective d'une tête de traitement et d'imagerie selon un quatrième mode de réalisation de l'invention,
La figure 9 est une vue schématique d'ensemble d'un appareil complet de traitement pouvant incorporer la tête d'imagerie et de traitement de l'invention,
La figure 10 est un organigramme montrant des différentes étapes du procédé de détermination de la distance réelle à la cible, et
Les figures 11a et 11b sont des vues schématiques en coupe transversale verticale de deux configurations de têtes qui diffèrent de par le positionnement de la sonde d'imagerie.

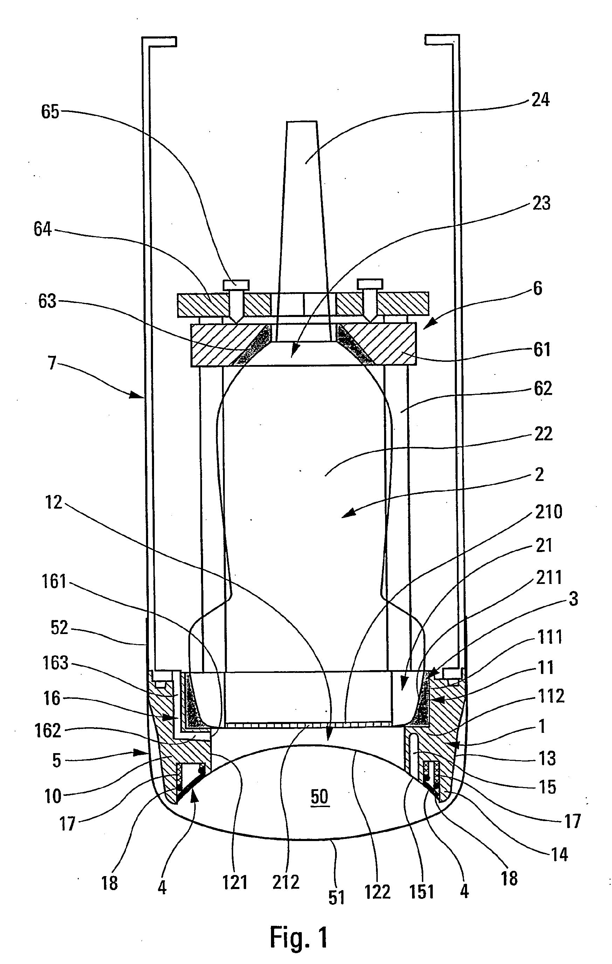

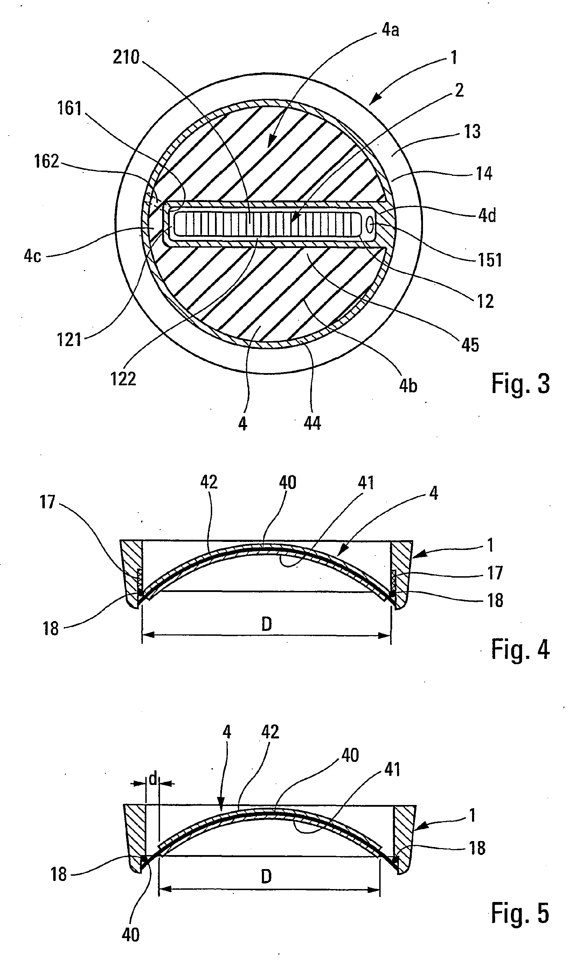

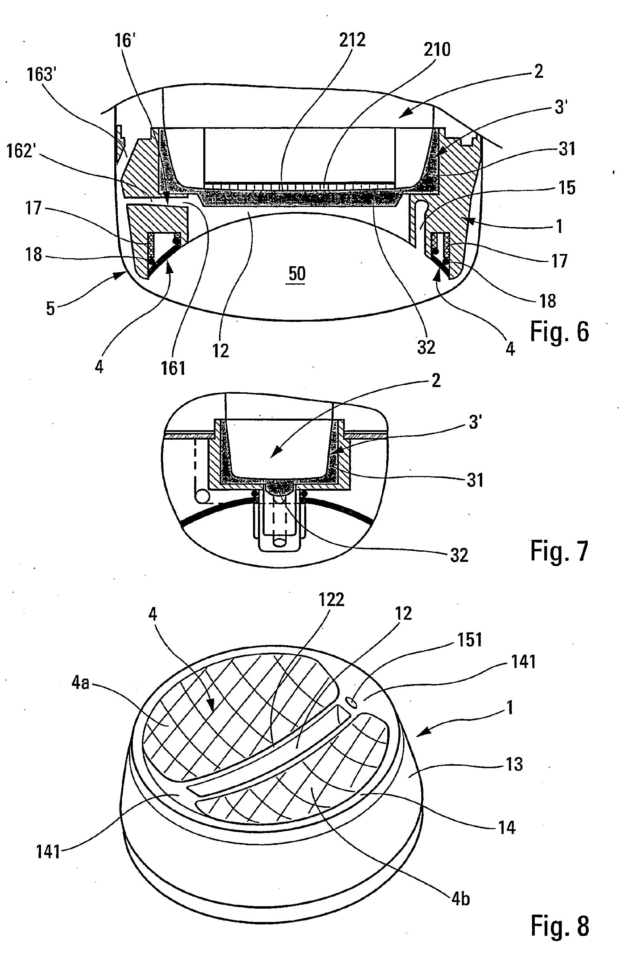

On se référera tout d'abord aux figures 1, 2 et 3 pour expliquer en détails la structure et le fonctionnement d'une tête d'imagerie et de traitement selon la première forme de réalisation de l'invention. La structure générale est reprise dans les troisième et quatrième formes de réalisation des figures 6, 7 et 8, alors que les formes de réalisation des figures 4 et 5 n'incorporent pas de moyens d'imagerie, uniquement des moyens de traitement.

La tête des figures 1 à 3 comprend sept éléments constitutifs, à savoir un corps de montage 1, des moyens d'imagerie 2, un adaptateur 3, des moyens de traitement 4, un ballonnet 5, un système de retenue 6 et un boîtier 7.

Les moyens d'imagerie 2 sont ici constitués par une sonde d'imagerie échographique, et on utilisera le terme « sonde » pour désigner les moyens d'imagerie dans le reste de la description. Il faut toutefois comprendre qu'une telle sonde n'est pas le seul dispositif utilisable en tant que moyen d'imagerie. On peut également utiliser des sondes à rayon X, ou des sondes IRM, cette énumération n'étant pas exhaustive. Tous moyens d'imagerie peuvent être utilisés.

Les moyens de traitement 4 sont ici constitués par un dispositif de thérapie qui peut utiliser des ultrasons focalisés ou non. La technique par ultrasons focalisés est conventionnellement désignée sous l'abréviation HIFU. Dans la suite de la description, les moyens de traitement seront désignés par le terme transducteur HIFU. Il faut cependant comprendre que ce transducteur HIFU n'est pas le seul dispositif utilisable en tant que moyen de traitement. On peut en effet utiliser n'importe quel dispositif invasif, ou de préférence non invasif, capable d'atteindre une cible située dans un organe ou un tissu d'un être vivant.

Le corps de montage 1 est une pièce de préférence monobloc, réalisée en n'importe quel matériau approprié comme de la matière plastique, du métal, de la céramique, ou des produits composites. De préférence, le corps 1 est réalisé par usinage ou moulage de métal, par exemple de l'aluminium ou de l'inox. Le corps de montage 1 constitue en quelques sortes la pièce centrale porteuse de la tête d'imagerie et de traitement selon cette forme de réalisation de l'invention. Le corps de montage 1 sert en effet au montage de la sonde échographique 2, du transducteur HIFU 4 et du ballonnet 5. Le corps 1 est monté sur le boîtier 7 qui sert à la fixation de la tête sur un bras articulé (non représenté). Le système de maintien 6 est également monté sur le corps 1 pour maintenir la sonde échographique 2 axialement en place sur le corps 1.

Le corps 1 présente une configuration générale globalement cylindrique circulaire. Le corps 1 comprend en effet une couronne 10 de forme globale cylindrique avec une paroi externe 13 sensiblement tronconique ou évasée. La couronne 10 comprend un talon d'ancrage 101 destiné à la fixation du corps 1 sur le boîtier 7. Le corps 1 comprend également un bord d'extrémité libre annulaire 14 à son extrémité opposée au talon d'ancrage 101. A l'intérieur de cette couronne 10, le corps 1 forme un logement de montage 11 destiné à la réception de la sonde échographique 2, comme on le verra ci-après. Ce logement 11 présente une forme allongée qui traverse diamétralement la couronne 10. La couronne 10 forme les deux extrémités du logement 11 qui sont reliées ensemble par deux parois longitudinales 110 qui traversent la couronne. Ce logement 11 présente une configuration sensiblement parallélépipédique, comme on peut le comprendre sur des figures 1, 2 et 3. Le logement 11 comprend une paroi latérale interne 111 qui s'étend de manière rectangulaire, et une paroi de fond 112. Avantageusement la paroi 111 est évasée vers le haut. La paroi 11 est de préférence garnie d'un matériau absorbant pour éviter les réflexions acoustiques qui dégraderaient autrement l'image. Cette paroi 112 est formée avec une ouverture qui donne dans une fenêtre 12, qui présente une configuration sensiblement similaire à celle du logement 11. En effet, la fenêtre 12 est allongée et s'étend de manière diamétrale d'un côté à l'autre de la couronne, comme on peut le voir sur la figure 1. La fenêtre 12 est bordée par des parois latérales 121 formées par la couronne et des parois latérales 122 qui traversent la couronne, comme on peut le voir sur la figure 3. Ainsi, le logement allongé 11 communique directement avec la fenêtre allongée 12. Plus précisément, la paroi de fond 112 du logement 11 se prolonge vers le bas par les parois latérales 121 et 122 de la fenêtre 12.

Au-delà de cette fenêtre 12, le corps 1 forme une zone de fixation destinée à la fixation du transducteur HIFU 4.

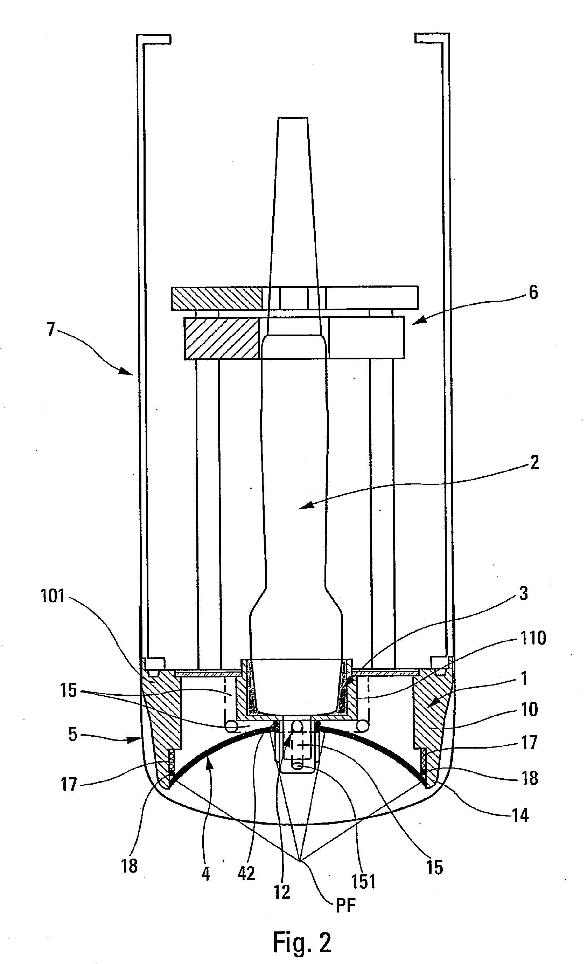

Cette zone de fixation présente une forme très complexe. En effet, cette zone de fixation s'étend tout d'abord sur tout ou partie du pourtour interne du bord d'extrémité libre 14 du corps 1. Cette zone s'étend aussi tout autour de la fenêtre 12, c'est-à-dire sur le bord des parois 121 et 122 de la fenêtre 12. Ceci est plus visible sur la figure 3. Comme on le verra ci-après, le transducteur HIFU 4 va s'étendre sur la totalité de l'étendue délimitée par le bord 14 à l'exception de la fenêtre 12. Ceci est visible sur la figure 3. Comme on peut le voir sur les figures 1 et 2, les bords des parois 122 sont réalisés de manière courbe concave, ce qui confère à l'ensemble de la tête une configuration concave. Le bord 14 est circulaire mais les bords des parois 122 sont concaves. De préférence, la concavité de la tête est de forme sphérique, avec un rayon de courbure déterminé, de sorte que les rayons convergent vers un point focal PF visible sur la figure 2. On peut même remarquer sur la figure 1 que la concavité des parois 122 est telle que la fenêtre 12 est réduite à un minimum au niveau de sa partie centrale. Le bord de la paroi 122 est à cet endroit très proche du logement 11.

Sur la figure 2, la fenêtre 12 a été partiellement représentée en perspective pour faire apparaître des caractéristiques qui ne sont pas situées dans le plan de coupe. Il s'agit plus particulièrement d'un canal d'alimentation en milieu de propagation qui est désigné par la référence numérique 15. Ce canal 15 débouche au niveau de la formation concave du corps 1 au niveau d'une entrée 151 située dans le prolongement de la fente 12, comme on peut le comprendre à partir des figures 1 et 3. Cette entrée 151 est formée au niveau de la couronne 14 à proximité de la zone de fixation du transducteur HIFU 4. Le canal d'alimentation 15 s'étend ensuite à l'intérieur du corps 1 en contournant le logement de montage 11. Ceci est visible sur la figure 2.

D'autre part, le corps de montage 1 forme également un canal d'évacuation de milieu de propagation qui est désigné par la référence numérique 16. Ce canal d'évacuation 16 comprend une sortie 161 qui débouche au niveau de la paroi latérale 121 de la fenêtre 12, à proximité directe du logement 11. Dans la forme de réalisation des figures 1, 2 et 3, ce canal d'évacuation 16 comprend une section radiale 162 qui s'étend en dessous du logement 11 et une section axiale 163 qui s'étend verticalement juste à côté du logement 11. La section 163 débouche ensuite au dessus du corps 1 où il peut être connecté à un tuyau d'évacuation. Il en est de même pour le canal 15 qui peut également être connecté à un tuyau d'alimentation. En variante représentée sur la figure 6, le canal d'évacuation 16' peut comprendre une section radiale 162 qui traverse la couronne 10 pour déboucher au niveau de la paroi externe 13. Ensuite, le milieu de propagation aspiré à travers la section radiale 162' peut s'écouler à travers une section sensiblement axiale 163' qui débouche au niveau du talon d'ancrage 101

La sonde échographique 2, qui fait ici fonction de moyen d'imagerie, est d'un modèle tout à fait classique disponible dans le commerce. La sonde échographique 2 est une sonde à barrettes linéaires 210. De manière typique, la sonde comprend une partie d'extrémité inférieure ou apicale 21, un corps 22 et une partie d'extrémité supérieure ou basale 23. La partie basale 23 est en outre pourvue d'un manchon de connexion 24 pour connecter la sonde à des moyens d'alimentation et de traitement de l'image prélevée. Les éléments linéaires 210 sont disposées côte à côte au niveau de la partie apicale 21. Les barrettes linéaires définissent une face d'émission et de réception d'ondes 212 qui présentent une configuration allongée rectangulaire. La partie apicale 21 définit autour de cette face 212 une paroi externe 211 de forme générale rectangulaire arrondie. Il s'agit là d'une forme particulière pour une sonde échographique : bien entendu on peut imaginer des sondes échographiques ayant d'autres formes. Toutefois, toutes les sondes comprennent une face d'émission d'ondes ou de rayonnements, une partie apicale et une partie basale.

La sonde échographique 2 est positionnée de manière précise, fixe et stable par rapport au corps 1 à l'aide de l'adaptateur 3. Plus précisément, la partie apicale 22 de la sonde échographique 2 est positionnée et maintenue à l'intérieur du logement 11 formé par le corps 1 par l'intermédiaire de l'adaptateur 3. Cet adaptateur 3 est de préférence réalisé en un matériau souple élastiquement déformable, comme un élastomère. L'adaptateur 3 permet de coupler la sonde échographique 2 au corps 1, en garantissant un positionnement précis de la face 212, et éventuellement une étanchéité au niveau du logement 11. L'adaptateur 3 est inséré dans le logement 11 en venant en contact à la fois de la paroi latérale 111 et de la paroi de fond 112. Le contact entre l'adaptateur 3 et le logement 11 est de préférence étanche aux fluides. Ainsi, l'adaptateur 3 présente une forme extérieure sensiblement rectangulaire allongée qui correspond à la forme du logement 11. Intérieurement, l'adaptateur 3 forme une paroi interne qui est adaptée en forme à la paroi externe périphérique 211 de la partie apicale 21. De préférence, un contact intime étanche est créé entre la paroi 211 et l'adaptateur 3. En résumé, l'adaptateur 3 présente une forme externe adaptée au logement 11 et une forme interne adaptée à la partie apicale de la sonde échographique. L'adaptateur 3 définit un passage allongé qui correspond environ à l'ouverture qui fait communiquer le logement 11 avec la fenêtre 12. On peut également dire que le passage formé par l'adaptateur 3 permet à la face d'émission 212 de la sonde échographique d'émettre à travers la fenêtre 12. Une autre définition serait que la face d'émission 212 obture la fenêtre 12 au niveau du logement 11. Ainsi, dans cette forme de réalisation, l'adaptateur 3 se présente sous la forme d'une bague de positionnement et d'étanchéité de forme générale torique rectangulaire avec un passage central pour recevoir la partie apicale 21 de la sonde échographique 2 de manière à ce que la face d'émission puisse émettre à travers la fenêtre 12. Dans cette forme de réalisation, l'adaptateur entoure ou ceinture la partie apicale 21 en laissant la face d'émission/réception 212 non revêtue de sorte qu'elle puisse émettre directement sans traverser l'adaptateur.

L'adaptateur 3 peut être fabriqué par n'importe quelle méthode ou technique de fabrication. On peut par exemple réaliser l'adaptateur de la manière suivante. On commence par prélever une empreinte numérisée de la partie apicale de la sonde échographique 2 afin d'obtenir une représentation géométrique et une estimation précise des dimensions de cette partie apicale. Ensuite, une partie de moule est réalisée en utilisant les dimensions prélevées lors de la numérisation de la partie apicale de la sonde. Cette partie de moule va définir le profil interne de l'adaptateur qui va recevoir la partie apicale de la sonde. De préférence, la dimension de moule est quelque peu réduite, de manière à ce que l'adaptateur soit légèrement plus petit que la partie apicale de la sonde. Ainsi, la partie apicale de la sonde sera reçue en force dans l'adaptateur en le déformant légèrement. Ceci assure non seulement un positionnement stable de la sonde dans l'adaptateur, mais également une parfaite étanchéité. En ce qui concerne la partie externe de l'adaptateur, elle est réalisée avec une autre partie de moule qui correspond exactement ou approximativement aux dimensions du logement 11 du corps 1. Ainsi, l'adaptateur est réalisé à l'aide de deux parties de moule, une partie correspond à la forme de la sonde 2, et l'autre partie correspondant à la forme du logement 11.

L'adaptateur 3 permet de positionner de manière précise, stable et étanche la partie apicale 21 de la sonde 2 sur le corps 1. Pour maintenir la sonde 2 de manière parfaitement axiale, il est en outre prévu un système de maintien 6 qui vient en prise avec la partie basale 23 de la sonde. Ce système de maintien 6 remplit ainsi une fonction de maintien axial et une fonction de poussée ou de sollicitation de la sonde dans l'adaptateur 3. Dans cette forme de réalisation non limitative, le système de maintien 6 comprend un étrier formé par des tiges longitudinales 62 et une platine fixe 64 pourvue de vis de poussée 65. Les tiges de coulissement 62 sont reliées au corps 1 par n'importe quel moyen. Une plaque de poussée 61 est montée coulissante sur les tiges 62 et peut être sollicitée en déplacement par les vis 65. La plaque 61 est avantageusement pourvue d'une bague de poussée 63 qui vient en contact avec la partie basale 23 de la sonde. Cette bague de poussée 63 peut être réalisée de la même manière que l'adaptateur 3. La partie externe de la bague de poussée 63 est adaptée à la forme sensiblement tronconique de la plaque de poussée 61. De manière symétrique, la face interne de la bague de poussée 63 est adaptée à la forme particulière de la partie basale 23. Ainsi, on assure un positionnement précis et stable de la partie basale 23 dans la plaque de poussée 61. En agissant sur les vis de poussée 65, la plaque de poussée 61 peut être déplacée en direction du corps 1, ce qui a pour effet de pousser la partie apicale 21 à l'intérieur de l'adaptateur 3 avec une force contrôlée. La bague de poussée 63 joue un rôle tout à fait symétrique à celui de l'adaptateur 3 au niveau de la plaque de poussée 61.

Le transducteur HIFU 4 est fixé sur le corps de montage 1 au niveau où il forme cette configuration concave, de préférence sphérique. Le transducteur 4 présente également une forme concave, de préférence sphérique. Comme on peut le voir sur la figure 3, le transducteur 4 s'étend à l'intérieur du bord 14 sur la totalité de l'étendue, excepté au niveau de la fenêtre 12. Dans le mode de réalisation de la figure 3, le transducteur 4 peut ainsi être divisé en quatre zones distinctes, à savoir une zone 4a située au dessus de la fenêtre 12, une zone 4b située en dessous de la fenêtre 12, une zone 4c à gauche de la fenêtre 12 et une zone 4d à droite de la fenêtre 12. On peut ainsi constater que le transducteur 4 entoure entièrement la fenêtre 12. On peut également constater que la fenêtre 12 coupe le transducteur 4 de manière sensiblement médiane, définissant ainsi les deux grandes zones 4a et 4b qui sont de dimension sensiblement identiques, et de forme également identiques par symétrie par rapport à la fenêtre 12. La symétrie est ici une symétrie miroir. Les zones 4c et 4d constituent des zones de jonction de part et d'autre de la fenêtre qui raccordent la zone 4a à la zone 4b. On peut également remarquer que le transducteur 4 ne s'étend pas sur l'entrée 151 du canal d'alimentation 15 en milieu de propagation. Sur la figure 3, on peut voir que le transducteur 4 comprend une zone de bord périphérique 44 et une zone de bord de fenêtre 45. Elles sont représentées de manière plus foncée que le restant du transducteur 4 qui est hachuré. Les bords 44, 45 et la zone 4d constituent les endroits par lesquels le transducteur 4 est fixé sur la zone de fixation du corps. Dans la variante de réalisation représentée sur la figure 8, le transducteur 4 ne comprend que deux zones 4a et 4b qui sont séparées l'une de l'autre. En effet, il n'y a pas de zone 4c et 4d dans ce mode de réalisation. A la place, le corps forme deux plages 141 qui relient le bord 14 à la fenêtre 12. L'entrée 151 est formée au niveau d'une des deux plages 141. Ainsi, le transducteur 4 ne s'étend que sur une partie du bord ou du pourtour.

On se référera maintenant aux figures 4 et 5 pour expliquer de quelle manière la surface utile active du transducteur 4 est étendue selon l'invention par rapport aux transducteurs de l'art antérieur. Sur la figure 5, on a représenté un transducteur de l'art antérieur monté sur un corps de montage d'une tête de traitement classique, qui n'incorpore pas de moyens d'imagerie. C'est pourquoi le transducteur 4 n'est pas interrompu par la fenêtre 12, comme c'est le cas de la tête d'imagerie et de traitement des figures 1 à 3. Dans cette forme de réalisation de l'art antérieur, le transducteur 4 comprend de manière conventionnelle un élément piézo-électrique 40 qui peut être réalisé à partir d'une seule ou de plusieurs pièces piézo-électriques. Cet élément piézo-électrique 40 présente une forme concave, de préférence sphérique. L'élément 40 peut être parfaitement circulaire ou présenter une autre forme quelconque. Pour amener l'élément piézo-électrique 40 en vibration, il est prévu une électrode active 42 qui s'étend sur la face convexe de l'élément 40. Sur sa face concave, l'élément 40 est pourvu d'une électrode de masse 41. Les électrodes 41 et 42 s'étendent sur la majeure partie de l'étendue de l'élément piézo-électrique 40, de manière à pouvoir l'exciter de manière maximale. L'élément piézo-électrique 40 est fixé au corps 1 au niveau d'une zone de fixation à l'aide d'un joint de colle 18. Avantageusement, le joint de colle 18 peut être logé dans une feuillure formée par le corps 1. Etant donné que le corps 1 est généralement réalisé en métal, comme de l'aluminium ou de l'inox, il n'est pas possible d'étendre l'électrode active 42 jusqu'en contact du corps 1. Il n'est même pas possible de se rapprocher de trop du corps 1, au risque de créer des arcs électriques. Par conséquent, il faut arrêter l'électrode active 42 à une distance minimale d du corps 1, comme on peut le voir sur la figure 5. Par conséquent, une zone de bord de l'élément piézo-électrique 40 n'est pas excitée, ce qui réduit considérablement la surface utile active du transducteur 4.

La figure 4 illustre de quelle manière la présente invention résout ce problème de réduction de la surface active du transducteur 4. Selon l'invention, il est prévu d'appliquer une garniture électriquement isolante 17 sur le corps 1 au niveau où l'électrode active 42 se rapproche du corps 1. Il est également possible d'appliquer une garniture isolante sur le transducteur, à la place ou en complément de la garniture isolante du corps. Le joint de colle 18 qui permet de fixer le transducteur 4 au corps 1 peut être appliqué sur le corps 1, ou avantageusement sur la garniture électriquement isolante 17. On peut ainsi étendre l'électrode active 42 jusqu'au niveau du joint de colle 18, sans risque de créer d'arc électrique, du fait de la présence de la garniture 17. Cette garniture 17 s'étend sur le corps 1 partout où le transducteur 4 est fixé au corps 1. Une telle garniture d'étanchéité 17 peut être utilisée sur n'importe quelle tête de traitement utilisant ou non des moyens d'imagerie. Sur la figure 4, la tête de traitement partiellement représentée n'incorpore pas de moyen d'imagerie. En revanche, dans le mode de réalisation des figures 1 à 3, la tête incorpore les moyens d'imagerie, et le transducteur 4 est fixé au corps 1 au moyen de joints de colle 18 associés à des garnitures d'étanchéité 17, visibles sur les figures 1 et 2. Ces garnitures électriquement isolantes 17 peuvent être réalisées en n'importe quel matériau approprié, comme par exemple des matériaux plastiques. On peut même prévoir de se servir de cette garniture d'étanchéité 17 pour réaliser la fixation du transducteur 4 sur le corps 1. Dans ce cas, la garniture se présente sous la forme d'une pièce sensiblement rigide dimensionnée avec précision et positionnée sur le corps de montage. Le transducteur est monté directement sur la garniture qui sert alors de moyens de support. La colle, si nécessaire, peut être appliquée sur la garniture. La garniture peut avoir la forme d'une bague rigide isolante adaptée à la fois à la forme du corps et du transducteur. La garniture peut être montée par surmoulage sur le corps. Grâce à cette garniture isolante 17, la surface active utile du transducteur 4 peut être augmentée de près de 20% par rapport à un transducteur de l'art antérieur, comme celui représenté sur la figure 5. L'utilisation de cette garniture isolante 17 est plus particulièrement préconisée au niveau du bord 14, étant donné que le gain de surface active y est plus important alors qu'il est plus faible au niveau de la fenêtre 12, en raison de sa position centrale.

Pour compléter la tête d'imagerie et de traitement du mode de réalisation des figures 1 et 2, le ballonnet 5, qui est réalisé en un matériau souple et de préférence élastiquement déformable, vient coiffer la tête 1. Le ballonnet 50 est fixé de manière étanche sur le corps 1 au niveau de son talon d'ancrage 101. Le transducteur 4 ainsi que la fenêtre 12 dans laquelle sont situées les barrettes 210, sont ainsi recouverts par le ballonnet 5. Etant donné que le transducteur 4 est fixé de manière étanche au corps 1 et que les barrettes 210 sont fixées de manière étanche dans le logement 110, il est créé avec le ballonnet 5 un espace interne de propagation 50 qui peut avantageusement être rempli d'un milieu de propagation des ondes émises par la sonde 2 et le transducteur 4. En pratique, le milieu de propagation peut-être de l'eau ou du gel échographique. L'espace de propagation 50 s'étend dans la forme concave du transducteur 4 et s'étend jusqu'au niveau de la paroi externe 13 du corps 1. Etant donné que le transducteur HIFU 4 génère de la chaleur qui est focalisée au niveau du point focal PF (figure 2), les ondes vont traverser le milieu de propagation situé dans l'espace 50. La chaleur produite par le transducteur 4 va alors chauffer le milieu de propagation. Pour éviter une élévation excessive de la température du milieu de propagation, il est connu de faire circuler le milieu de propagation à l'intérieur de l'espace de propagation 50. Pour cela, la tête est pourvue d'une alimentation en eau et d'une évacuation en eau. Plus précisément, comme susmentionné, le corps 1 forme un canal d'alimentation 15 et un canal d'évacuation 16 ou 16'. L'eau pénètre dans l'espace 50 par le canal d'alimentation 15 et sort de cet espace par le conduit d'évacuation 16 ou 16'. On assure ainsi une température constante et relativement basse du milieu de propagation. En outre, afin d'éviter la formation de bulles à l'intérieur de l'espace de propagation, l'invention prévoit de situer la sortie 161 du canal d'évacuation 16 ou 16' au niveau de la fenêtre 12, et de préférence, le plus près possible de la face d'émission 212 de la sonde. En effet, on a constaté de manière empirique que les bulles ont tendance à s'accumuler au niveau de la face d'émission de la sonde et ainsi créer un voile au travers duquel la sonde ne peut pas émettre sans diffractions gênantes. En positionnant la sortie 161 le plus haut possible à proximité de la face d'émission, on évite la formation d'un tel voile de bulles.

On se référera maintenant aux figures 6 et 7 pour expliquer une variante de réalisation avantageuse de l'adaptateur. Alors que l'adaptateur 3 des figures 1 et 2 ne forment qu'une bague d'étanchéité et de positionnement engagée autour de la sonde en laissant la face d'émission découverte, l'adaptateur 3' des figures 6 et 7 comprend une paroi frontale 32 qui s'étend devant la face d'émission de la sonde et qui obture en même temps l'ouverture entre le logement 11 et la fenêtre 12. Ainsi, la face d'émission de la sonde n'est plus en contact avec le milieu de propagation présent dans l'espace de propagation 50. L'adaptateur 3' forme ainsi une sorte d'enveloppe qui entoure la totalité de la partie inférieure de la sonde. Avantageusement, cette paroi frontale 32 est réalisée de telle sorte qu'elle forme une lentille divergente disposée devant la face d'émission de la sonde afin d'éloigner ou de déplacer le point focal des ondes émises par la sonde d'une distance de quelques centimètres, avantageusement deux cm. Alors que le point focal de la sonde échographique se situerait normalement au niveau du ballonnet 5, la lentille divergente 32 permet de déplacer ce point focal vers l'extérieur du ballonnet d'une distance de quelques centimètres, afin de pouvoir donner une représentation fiable d'un organe ou tissu d'un être vivant situé à deux centimètres sous sa peau. L'adaptateur 3' doit par conséquent être réalisé en un matériau approprié, permettant le passage des ultrasons générés par la sonde.

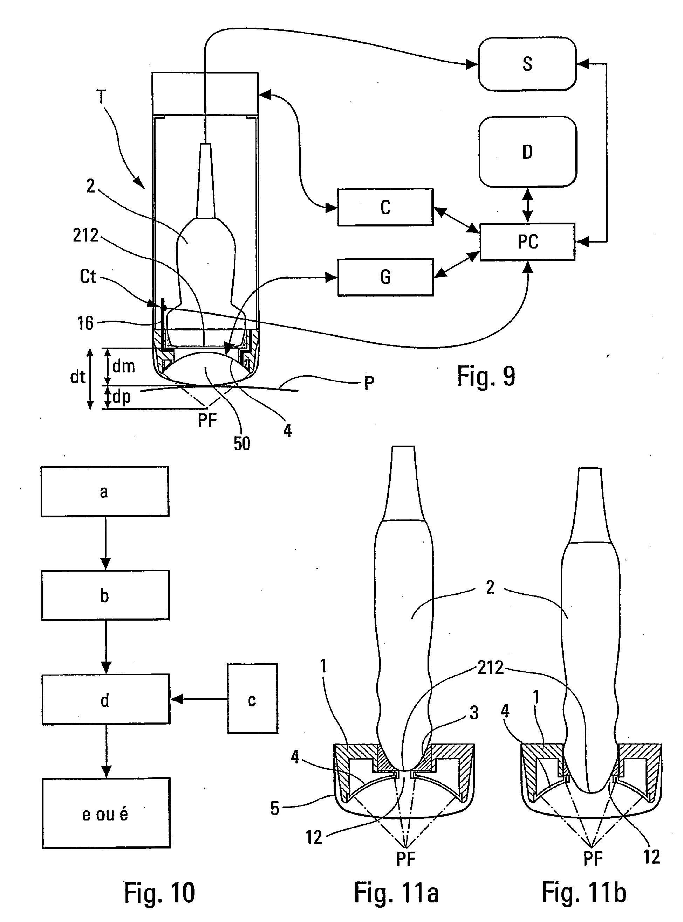

Un autre caractéristique intéressante de l'invention réside dans le fait que la sonde s'étend de manière sensiblement médiane par rapport au transducteur en le divisant ainsi en deux parties sensiblement identiques par symétrie miroir. Plus précisément, la fente 12 qui reçoit la face 212 coupe le transducteur en deux. Il est particulièrement avantageux d'utiliser une sonde à barrettes linéaires ou planes avec cette configuration. On peut aussi faire varier facilement la position de la face 212 par rapport au transducteur en élargissant ou en rétrécissant la largeur le la fente 12, comme on peut le comprendre à partir des figures 11a et 11b.

La tête ou les têtes qui viennent d'être décrites peuvent être incorporées dans un appareil de traitement tel que représenté schématiquement sur la figure 9. Dans cet appareil, la tête T est associée à plusieurs éléments, comme par exemple un générateur de puissance G pour alimenter les moyens de traitement (transducteur 4), un contrôleur de déplacement C pour déplacer la tête, un scanner à ultrasons S relié à la sonde 2, un écran de visualisation D et un ordinateur PC qui sert à gérer l'appareil. Tous les éléments de l'appareil sont reliés au PC. L'écran D sert à visualise le site cible prélevé par la sonde de la tête reliée au scanner S. En outre, le canal d'évacuation 16 de la tête est pourvu d'un capteur de température Ct apte à mesurer la température du milieu de propagation qui quitte l'espace de propagation. La mesure est directement envoyée au PC. L'utilisation de cette mesure de température sera donnée ci-après. La face d'émission/réception de la tête émet des ondes qui traversent d'abord le milieu de propagation sur un certaine épaisseur dm et le tissu vivant sur un autre épaisseur dp, soit une épaisseur totale cumulée dt.

A cet égard, il existe un problème de détermination de la distance qui sépare la face d'émission/réception de la sonde et le site cible à visualiser, et ensuite à traiter. En effet, la sonde échographique est éloignée du patient, et les ultrasons émis et reçus par la sonde échographique se propagent dans le liquide de propagation réfrigéré sur une distance importante. Or on sait que la vitesse des ultrasons dépend de la température et de la nature du milieu. En revanche, les échographes sont réglés sur le principe que les ultrasons se propagent dans le tissu du patient, et calculent donc la distance à la cible à partir de la vitesse dans un tissu typique, qu'ils supposent être à 37°C. Mais dans le cas d'une tête d'imagerie, qui incorpore en outre des moyens de traitement, les ondes émises par la sonde doivent traverser une certaine épaisseur de milieu de propagation, qui est maintenu avantageusement à une température constante de l'ordre de 10°C environ. Par conséquent, non seulement les ondes doivent parcourir une distance plus grande que celle d'une sonde appliquée à même la peau du patient, mais elles traversent en outre un milieu qui est différent du tissu vivant, aussi bien en nature qu'en température. La traversée du milieu de propagation induit donc une aberration dans le calcul de la distance à la cible qu'il vaut mieux corriger pour pouvoir effectuer un traitement précis et efficace.

En effet, le son se propage plus lentement dans le milieu de propagation que dans le tissu. L'échographe traduit cette durée de parcours accru comme une distance plus grande, et la cible paraît donc plus loin qu'elle ne l'est réellement. L'exemple donné montre que cet écart peut être significatif, en particulier pour les systèmes très focalisés dans lesquels la position du point focal est très précise.

Or, le point de concentration des ultrasons thérapeutiques émis par les moyens de traitement n'est généralement pas, ou peu modifié par la température du milieu. En effet les ultrasons émis par les moyens de traitement, par exemple un transducteur 4, se concentreront toujours au centre de la sphère. Dans un système différent, l'effet des ultrasons peut être modifié par la température, et il conviendra alors d'en tenir compte.

L'organigramme représenté sur la figure 10 illustre schématiquement par des blocs les différentes étapes du procédé de détermination de la distance réelle séparant la face 212 de la cible. On commence a) par mesurer la température du milieu de propagation à l'aide du capteur Ct. Ensuite, en b) on calcule la vitesse de propagation des ondes de la sonde dans le milieu de propagation à la température mesurée en a). On détermine ou on connaît en c) l'épaisseur du milieu de propagation traversée par les ondes. On peut ainsi calculer en d) l'erreur ou correction de distance.

Supposons par exemple, que :
- la cible se trouve au point focal du transducteur 4, à 40 mm de la face d'émission/réception de la sonde.
- la cible se trouve à dp = 15mm sous la peau. Les ondes doivent donc traverser une épaisseur dm = 25 mm de milieu de propagation.
- le milieu de propagation est de l'eau réfrigérée à 10°C dont la vitesse est 1449 m/s. Cette vitesse peut être déterminée à partir de tables de valeurs préétablies en fonction de la nature du milieu de propagation, typiquement de l'eau.
- La correction de distance peut alors être déterminée à l'aide d'un logiciel chargé dans le PC. La correction est égale à 1,2 mm.

On peut imputer cette correction de distance de plusieurs façons comme l'indique la figure 10. Par exemple dans l'appareil de la figure 9, l'image échographique provenant de la sonde est reproduite sur l'écran D de l'ordinateur PC sur lequel on représente également la position du point focal par une marque. Une première correction peut consister à modifier la position de la marque du point focal de la distance calculée .Dans le cas présent, la sonde donnera la position de la cible à 41,2 mm de la face 212 et il faudra donc rapprocher la marque du point focal de 1,2mm vers le début de l'image échographique. Une deuxième correction peut consister simplement à déplacer lors du tir la tête de traitement suivant son axe (dans le cas présent un retrait de 1,2mm). Ces deux possibilités d'appliquer la correction distance sont représentées en e et e' sur la figure 10. Une troisième correction peut consister à modifier les paramètres de l'échographe, par exemple sa valeur interne de la vitesse du son dans les tissus.

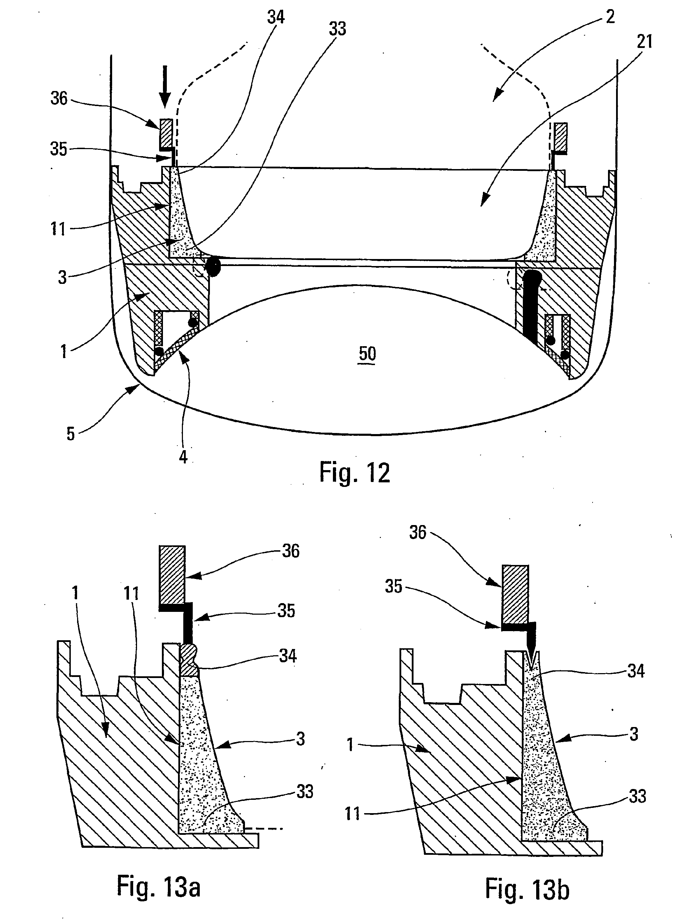

En se référant maintenant aux figures 12, 13a et 13b, on voit que l'adaptateur 3 peut être comprimé, déformé ou dilaté latéralement dans son logement 11 par une douille de compression 35 qui est elle-même sollicitée par un pièce de poussée 36 qui exerce une force sur la douille dans le sens de la flèche représentée sur la figure 12. L'adaptateur 3 comprend une partie inférieure 33 qui est plus épaisse, et de ce fait plus rigide et plus stable en dimension. A l'opposé, l'adaptateur comprend une partie supérieure 34 qui est plus mince, et de ce fait déformable. La douille appuie sur l'adaptateur au niveau de la partie supérieure déformable. Ainsi, la partie supérieure va se déformer par écrasement axial et dilatation latérale de manière à appuyer, avantageusement de manière étanche, contre la partie apicale 21 de la sonde 2. La partie inférieure rigide reste sensiblement non déformée et assure ainsi le positionnement précis et stable de la sonde. La douille appuie sur l'adaptateur tout autour de la sonde, de préférence de manière régulière.

En regardant la figure 13a, on peut remarquer que l'adaptateur 3 peut être réalisé de manière monobloc, par exemple par bi-injection ou surmoulage, avec deux matériaux différents, à savoir un premier matériau sensiblement rigide pour former la partie inférieure 33 et un second matériau déformable pour former la partie supérieure 34. Dans cette forme de réalisation de la figure 13a, la partie supérieure 34 forme un cordon périphérique qui va être écrasé par la douille 35 pour appuyer à la fois contre le logement 11 et la sonde 2.

Dans la forme de réalisation de la figure 13b, l'adaptateur est moulé dans un matériau unique. Sa partie supérieure 34 forme une gorge périphérique dans lequel la douille est appuyée de manière à élargir la gorge et pousser ses parois latérales respectivement vers le logement et vers la sonde.

L'action de la douille sur l'adaptateur engendre ainsi une déformation au moins locale ou partielle de l'adaptateur qui se dilate latéralement.

Bien que la présente invention ait été décrite en référence à une tête d'imagerie et de traitement incorporant à la fois des moyens d'imagerie et des moyens de traitement, on comprendra aisément que certaines caractéristiques, notamment la garniture isolante 17, peuvent être mises en oeuvre sur d'autres types de têtes de traitement, qui n'incluent pas forcément des moyens d'imagerie. Les diverses caractéristiques inventives permettent de réaliser une tête thérapeutique précise, efficace, et puissante. En effet, l'adaptateur permet un positionnement amovible mais précis des moyens d'imagerie, la position du canal d'évacuation assure un débullage efficace et la garniture isolante permet d'augmenter considérablement la puissance des moyens de traitement. Une telle tête permet de traiter les tumeurs, les concrétions, les os ou plus généralement tout organe d'un être vivant.

## Revendications

1. Tête d'imagerie et de traitement d'organe ou de tissu d'être vivant, notamment utile pour le traitement de tumeurs prostatiques ou thyroïdiennes, ladite tête comportant :
- des moyens d'imagerie (2), tels qu'une sonde échographique, aptes à émettre des ondes ou des rayonnements pour fournir une représentation imagée d'un tissu ou organe à traiter,
- des moyens de traitement (4) aptes à traiter localement ledit tissu ou organe, les moyens de traitement étant aptes à émettre des ondes ou des rayonnements pour traiter le tissu ou l'organe,
dans laquelle les moyens d'imagerie (2) sont montés de manière amovible sur un adaptateur (3 ; 3'), assurant le positionnement des moyens d'imagerie (2) relativement aux moyens de traitement (4), la tête comportant un corps de montage (1) comprenant une zone de fixation des moyens de traitement et un logement de montage (11) des moyens d'imagerie (2), ledit logement de montage (11) recevant ledit adaptateur (3 ; 3') dans lequel les moyens d'imagerie sont montés de manière amovible,
caractériséeen ce que ledit adaptateur (3 ; 3') comprend une bague d'étanchéité souple déformable, assurant l'étanchéité entre les moyens d'imagerie et le logement de montage, les moyens d'imagerie (2) déformant la bague lors de leur insertion dans la bague.

2. Tête selon la revendication 1, dans laquelle l'adaptateur (3 ; 3') présente une forme externe adaptée au logement (11) et une forme interne adaptée aux moyens d'imagerie (2).

3. Tête selon la revendication 1 ou 2, comprenant un espace de propagation (50) contenant un milieu de propagation apte à transmettre les ondes ou rayonnements émis(es) par les moyens d'imagerie et/ou les moyens de traitement, le corps (1) formant une fenêtre (12) qui fait communiquer le logement (11) avec l'espace de propagation (50), lesdits moyens d'imagerie (2) comportant une partie apicale (21) et une partie basale (23), ladite partie apicale (21) incorporant une face d'émission (212) d'ondes ou de rayonnement, reçue dans l'adaptateur (3 ; 3') de manière à émettre à travers la fenêtre (12) dans l'espace de propagation (50).

4. Tête selon la revendication 3, dans laquelle ledit adaptateur (3') comporte une paroi frontale (32) positionnée devant la face d'émission (212) des moyens d'imagerie et obturant au moins partiellement la fenêtre (12), ladite paroi frontale (32) formant une lentille divergente, apte à éloigner le point focal (PF) des ondes ou rayonnements émis à travers la lentille et l'espace de propagation.

5. Tête selon l'une quelconque des revendications précédentes, comprenant en outre une douille de compression qui déforme au moins partiellement l'adaptateur dans son logement pour l'appuyer de manière étanche contre les moyens d'imagerie.

6. Tête selon la revendication 5, dans laquelle l'adaptateur comprend une partie inférieure sensiblement indéformable et stable dimensionnellement et une partie supérieure déformable, la partie inférieure assurant le positionnement précis des moyens d'imagerie et la partie supérieure assurant l'étanchéité, les parties inférieures et supérieures étant avantageusement réalisées avec des matériaux différents.

7. Tête selon l'une quelconque des revendications précédentes, dans laquelle lesdits moyens d'imagerie (2) sont maintenus en position dans ledit adaptateur (3 ; 3') au moyen d'un système de maintien (6) apte à solliciter les moyens d'imagerie dans ledit adaptateur.

8. Tête selon l'une quelconque des revendications précédentes, dans laquelle lesdits moyens d'imagerie comportent une sonde échographique à barrettes linéaires (210).

9. Tête selon la revendication 6, dans laquelle le corps forme une fenêtre allongée (12), les barrettes linéaires (210) étant positionnées pour émettre à travers la fenêtre, les moyens de traitement (4) étant positionnés de part et d'autre de la fenêtre allongée (12).

10. Tête selon l'une quelconque des revendications précédentes, dans laquelle lesdits moyens de traitement (4) comportent un transducteur piézo-électrique focalisé du type HIFU.

11. Procédé de fabrication d'un adaptateur (3 ; 3') pour des moyens d'imagerie (2) incorporés dans une tête d'imagerie et de traitement focalisés selon l'une quelconque des revendications précédentes, dans lequel ledit procédé comprend les étapes de :
(a) fournir des moyens d'imagerie comportant une partie apicale comprenant une face d'émission servant à la transmission d'ondes ou de rayonnement,
(b) numériser ladite paroi apicale des moyens d'imagerie afin d'obtenir une représentation géométrique et une estimation précise des dimensions de cette partie apicale,
(c) fournir un moule présentant une géométrie et des dimensions en accord avec le résultat obtenu à l'étape (b),
(d) couler un ou plusieurs matériaux élastomériques dans le moule, de manière à obtenir un produit de moulage formant un adaptateur apte à parfaitement se positionner sur la partie apicale des moyens d'imagerie.

## Claims

1. An imaging and treatment head for imaging and treating an organ or tissue of a living being, and usable in particular for treating tumors of the prostate or the thyroid, said head comprising:
• imaging means (2), such as an ultrasonic probe, that are suitable for emitting waves or radiation so as to provide an imaged representation of an organ or tissue for treatment; and
treatment means (4) that are suitable for treating said organ or tissue locally, the treatment means being suitable for emitting waves or radiation so as to treat the organ or the tissue;
wherein the imaging means (2) are mounted in removable manner on an adapter (3; 3') that ensures positioning of the imaging means (2) relative to the treatment means (4), the head including a mounting body (1) including a fastener zone for fastening the treatment means, and a mounting housing (11) for mounting the imaging means (2), said mounting housing (11) receiving said adapter (3; 3') in which the imaging means are mounted in removable manner,
said head being **characterized in that** said adapter (3; 3') includes a flexible deformable sealing ring that ensures sealing between the imaging means and the mounting housing, the imaging means (2) deforming the ring while they are being inserted into the ring.

2. A head according to claim 1, wherein the adapter (3; 3') has an outer shape adapted to the housing (11) and an inner shape adapted to the imaging means (2).

3. A head according to claim 1 or 2, including a propagation space (50) containing a propagation medium that is suitable for transmitting the waves or radiation emitted by the imaging means and/or the treatment means, the body (1) forming a window (12) that puts the housing (11) into communication with the propagation space (50), said imaging means (2) including a tip portion (21) and a base portion (23), said tip portion (21) incorporating an emitter face (212) for emitting waves or radiation, and for being received in the adapter (3; 3') in such a manner as to emit through the window (12) in the propagation space (50).

4. A head according to claim 3, in which said adapter (3') includes a front wall (32) that is positioned in front of the emitter face (212) of the imaging means and that closes the window (12) at least in part, said front wall (32) forming a diverging lens that is suitable for distancing the focal point (FP) of the waves or radiation emitted through the lens and the propagation space.

5. A head according to any preceding claim, further including a compression bushing that deforms the adapter, at least in part, in its housing, so as to press it in leaktight manner against the imaging means.

6. A head according to claim 5, in which the adapter includes a bottom portion that is substantially non-deformable and dimensionally stable, and a top portion that is deformable, the bottom portion ensuring accurate positioning of the imaging means, and the top portion ensuring sealing, the bottom and top portions advantageously being made of different materials.

7. A head according to any preceding claim, in which said imaging means (2) are held in position in said adapter (3; 3') by means of a holding system (6) that is suitable for urging the imaging means into said adapter.

8. A head according to any preceding claim, in which said imaging means comprise an ultrasonic probe having a linear array (210).

9. A head according to claim 6, in which the body forms an elongate window (12), the linear array (210) being positioned so as to emit through the window, the treatment means (4) being positioned on either side of the elongate window (12).

10. A head according to any preceding claim, in which said treatment means (4) comprise a focused piezoelectric transducer of the HIFU type.

11. A method of manufacturing an adapter (3; 3') for imaging means (2) incorporated in a focused-imaging/treatment head according to any preceding claim, in which said method comprises the steps of:
a) providing imaging means that include a tip portion that includes an emitter face through which waves or radiation are transmitted;
b) digitally scanning said tip portion of the imaging means in order to obtain a geometrical representation and an accurate estimation of the dimensions of said tip portion;
c) providing a mold presenting a shape and dimensions that match the result obtained in step b); and
d) pouring one or more elastomeric materials into the mold so as to obtain a molded product that forms an adapter that is suitable for being positioned accurately on the tip portion of the imaging means.

## Patentansprüche

1. Kopf zur bildlichen Darstellung und Behandlung von Organen oder Gewebe eines Lebewesens, insbesondere für einen Einsatz zur Behandlung von Prostata- und Schilddrüsentumoren, wobei der Kopf aufweist:
- Bilddarstellungsmittel (2), wie etwa eine Echographiesonde, die dazu ausgelegt sind, für eine Bildwiedergabe von zu behandelndem Gewebe oder ein zu behandelndes Organ Wellen oder Strahlung auszusenden,
- Behandlungsmittel (4), die zur lokalen Behandlung des Gewebes oder Organs ausgelegt sind, wobei die Behandlungsmittel zur Behandlung des Gewebes oder des Organs zur Aussendung von Wellen oder Strahlung ausgelegt sind,
wobei die Bilddarstellungsmittel (2) zur Gewährleistung der Positionierung der Bilddarstellungsmittel (2) relativ zu den Behandlungsmitteln abnehmbar auf einem Adapter (3; 3') angebracht sind, wobei der Kopf einen Montagekörper (1) aufweist, wobei der Kopf einen Montagekörper (1) aufweist, der eine Befestigungszone für die Behandlungsmittel und eine Montageaufnahme (11) für die Bilddarstellungsmittel (2) aufweist, wobei die Montageaufnahme (11) den Adapter (3,3') aufnimmt, in dem die Bilddarstellungsmittel abnehmbar angebracht sind;
**dadurch gekennzeichnet, dass** der Adapter (3, 3') einen nachgiebigen, verformbaren Dichtungsring aufweist, der die Dichtigkeit zwischen den Bilddarstellungsmitteln und der Montageaufnahme gewährleistet, wobei die Bilddarstellungsmittel (2) den Ring bei ihrer Einführung in diesen verformen.

2. Kopf nach Anspruch 1, wobei der Adapter (3,3') eine an die Aufnahme (11) angepasste äußere Gestalt und eine an die Bilddarstellungsmittel (2) angepasste innere Gestalt aufweist.

3. Kopf nach Anspruch 1 oder 2, aufweisend einen Ausbreitungsraum (50), der ein Ausbreitungsmedium enthält, das geeignet ist, die Wellen oder die Strahlung zu übertragen, die durch die Bilddarstellungsmittel und/oder die Behandlungsmittel ausgestrahlt werden bzw. wird, wobei der Körper (1) ein Fenster (12) bildet, das die Aufnahme (11) mit dem Ausbreitungsraum (50) verbindet, wobei die Bilddarstellungsmittel (2) einen apikalen Teil (21) und einen basalen Teil (23) aufweisen, wobei der apikale Teil (21) eine Wellen- oder Strahlungsaussendefläche (212) aufweist, die in dem Adapter (3,3') derart aufgenommen ist, dass sie die Wellen bzw. die Strahlung durch das Fenster hindurch in den Ausbreitungsraum aussendet.

4. Kopf nach Anspruch 3, wobei der Adapter (3') eine vor der Aussendefläche (212) der Bilddarstellungsmittel angeordnete und das Fenster (12) zumindest teilweise verschließende Vorderwand (32) aufweist, wobei die Vorderwand (32) eine Zerstreuungslinse bildet, die dazu ausgelegt ist, den Brennpunkt (PF) der Wellen oder Strahlung, die durch die Linse und den Ausbreitungsraum ausgestrahlt werden bzw. wird, weiter weg zu verlegen.

5. Kopf nach einem der vorangehenden Ansprüche, außerdem aufweisend eine Kompressionshülse, die den Adapter zur dichten Anlage an den Bilddarstellungsmitteln in seiner Aufnahme zumindest teilweise verformt.

6. Kopf nach Anspruch 5, wobei der Adapter einen im wesentlichen unverformbaren und bezüglich seiner Abmessung stabilen unteren Teil und einen verformbaren oberen Teil aufweist, wobei der untere Teil die präzise Positionierung der Bilddarstellungsmittel und der obere Teil die Dichtigkeit gewährleistet, wobei die oberen und unteren Teile bevorzugt aus unterschiedlichen Materialien bestehen.

7. Kopf nach einem der vorangehenden Ansprüche, wobei die Bilddarstellungsmittel (2) in ihrem Adapter (3; 3') mittels eines Haltesystems (6) in Position gehalten sind, das dazu ausgelegt ist, die Bilddarstellungsmittel in dem Adapter vorzuspannen.

8. Kopf nach einem der vorangehenden Ansprüche, wobei die Bilddarstellungsmittel eine Echographiesonde mit linearen Elementen (210) aufweisen.

9. Kopf nach Anspruch 8, wobei der Körper ein langgestrecktes Fenster (12) bildet, wobei die linearen Elemente (210) so angeordnet sind, dass sie durch das Fenster hindurch aussenden, wobei die Behandlungsmittel (4) beiderseits des langgestreckten Fensters (12) zu liegen kommen.

10. Kopf nach einem der vorangehenden Ansprüche, wobei die Behandlungsmittel (4) einen fokussierten piezoelektrischen HIFU-Wandler aufweisen.

11. Verfahren zur Herstellung eines Adapters (3; 3') für die Bilddarstellungsmittel (2), die in einem Kopf zur fokussierten bildlichen Darstellung und Behandlung nach einem der vorangehenden Ansprüche enthalten sind, wobei das Verfahren die Schritte aufweist:
a) Bereitstellen von Bilddarstellungsmitteln, die einen apikalen Teil aufweisen, der eine Aussendefläche zur Übertragung von Wellen oder Strahlung aufweist,
(b) Digitalisieren der apikalen Wand der Bilddarstellungsmittel zur Gewinnung einer geometrischen Darstellung und einer präzisen Erfassung der Abmessung dieses apikalen Teils,
(c) Bereitstellen einer Abformung entsprechend einer Geometrie und Abmessung, die im Schritt (b) gewonnen wurden,
(d) Abkühlen von einem Elastomermaterial oder mehreren derartigen Materialen zum Bereitstellen eines Abformungserzeugnisses, das einen Adapter bildet, der dazu ausgelegt ist, perfekt auf den apikalen Teil der Bilddarstellungsmittel zu passen.
